# FASCICULE DE BREVET EUROPEEN

(11) **EP 3 478 815 B1**
(45) Date de publication et mention de la délivrance du brevet: **27.11.2024**
(21) Numéro de dépôt: 17723132.1
(22) Date de dépôt: 17.05.2017
(51) Int. Cl.: C12F 3/04, C12P 7/16

(54) **PROCEDE DE RECUPERATION D'ALCOOLS DANS UN FERMENTEUR**
VERFAHREN ZUR RÜCKGEWINNUNG VON ALKOHOLEN IN EINEM FERMENTER
PROCESS FOR THE RECOVERY OF ALCOHOLS IN A FERMENTER

(30) Priorité: 30.06.2016 FR 1656209
(43) Date de publication de la demande: 08.05.2019
(73) Titulaire: IFP Energies nouvelles, 92852 Rueil-Malmaison (FR)
(72) Inventeur: COUPARD, Vincent, 69100 Villeurbanne (FR); GONZALEZ PEÑAS, Helena, 69007 Lyon (FR); TOTH, Eszter, 69008 Lyon (FR); LE MOEL, Mehdi, 69600 Oullins (FR)
(74) Mandataire: IFP Energies nouvelles
(86) Numéro de dépôt international: PCT/EP2017/061801
(87) Numéro de publication internationale: WO 2018/001628

(56) Documents cités:
- FR-A1- 2 974 116
- FR-A1- 3 023 300
- CHUANG XUE ET AL: "High-titer n -butanol production by clostridium acetobutylicum JB200 in fed-batch fermentation with intermittent gas stripping", BIOTECHNOLOGY AND BIOENGINEERING, vol. 109, no. 11, 8 November 2012 (2012-11-08), pages 2746 - 2756, XP055346003, ISSN: 0006-3592, DOI: 10.1002/bit.24563
- HANNO RICHTER ET AL: "Prolonged conversion of n-butyrate to n-butanol with Clostridium saccharoperbutylacetonicum in a two-stage continuous culture with in-situ product removal", BIOTECHNOLOGY AND BIOENGINEERING, vol. 109, no. 4, 17 November 2011 (2011-11-17), pages 913 - 921, XP055081864, ISSN: 0006-3592, DOI: 10.1002/bit.24380
- THADDEUS C EZEJI ET AL: "Improving performance of a gas stripping-based recovery system to remove butanol from Clostridium beijerinckii fermentation", BIOPROCESS AND BIOSYSTEMS ENGINEERING, SPRINGER, BERLIN, DE, vol. 27, no. 3, 2 April 2005 (2005-04-02), pages 207 - 214, XP019347339, ISSN: 1615-7605, DOI: 10.1007/S00449-005-0403-7

## Description

La présente invention concerne un procédé de récupération de produits de fermentation d'un jus de fermentation contenu dans un fermenteur. Le procédé selon l'invention est particulièrement adapté pour récupérer des alcools, des esters, des acides carboxyliques et des cétones, des aldéhydes produits par fermentation d'une solution aqueuse de sucres en C5 et/ou C6. Le procédé de récupération peut être notamment appliqué à un procédé de fermentation du type ABE (Acétone/Butanol/Ethanol) ou IBE (Isopropanol/Butanol/Ethanol).

### Etat de la technique

Afin de répondre aux enjeux de la transition énergétique, de nombreuses recherches sont menées pour développer des procédés dits «verts», permettant d'accéder à des intermédiaires chimiques d'une façon alternative au raffinage du pétrole et/ou à la pétrochimie.

Les alcools issus de fermentation (n-butanol, isopropanol) sont les substituts de dérivés pétrochimiques les plus prometteurs. La fermentation ABE (Acétone - Butanol - Ethanol) est une des plus anciennes fermentations à avoir été industrialisée (début du 20ème siècle) et a été depuis largement étudiée. On peut également citer la fermentation IBE produisant un mélange d'isopropanol, butanol et éthanol. Ces deux types de fermentations sont réalisées en anaérobiose stricte en présence d'un microorganisme fermentaire généralement du genre *Clostridium.*

Un des verrous dans le développement de procédés fermentaires est l'étape de récupération des produits du milieu aqueux fortement dilué. C'est le paramètre déterminant au niveau de l'économie de ces types de procédé. Afin de rendre la production fermentaire à grande échelle économiquement viable, il s'avère nécessaire de maximiser le titre final ainsi que la productivité volumique dans le bioréacteur, ces deux paramètres étant fortement limités par l'inhibition marquée des microorganismes vis-à-vis des produits d'intérêt. Il est en effet connu que le butanol à partir d'une concentration dans le milieu fermentaire a un effet inhibiteur sur le microorganisme par exemple chez *Clostridium.* L'usage de techniques de couplage fermentation - séparation (extraction liquide, adsorption, stripage...) permet la récupération des produits inhibiteurs au fur et à mesure qu'ils sont produits. Ces techniques offrent des moyens de franchir la limite d'inhibition imposée durant la production microbienne, et par conséquence d'alléger la demande énergétique requise pour la distillation d'un jus de fermentation final extrêmement dilué.

Les techniques de récupération in situ du butanol ont été largement explorées dans la littérature pour la fermentation Acétone/Butanol/Ethanol.

La récupération des produits issus d'une fermentation ABE par stripage avec un gaz injecté dans le bioréacteur a été proposée dans la littérature (Quereshi et Blaschek (Renewable Energy, 22 (4)) ou Ezeji et al. (Appl. Microbio. Biotechnol., 63 (6)). Dans ces schémas, les alcools sont récupérés par condensation du gaz issu du réacteur, ce qui s'avère extrêmement couteux du point de vue énergétique.

Kuan-Ming & al. (Journal of the Taiwan Institute of Chemical Engineers, 45 (2014) 2106-2110) décrivent un procédé intégré couplant stripage au gaz et extraction liquide-liquide avec l'oleyl alcool *in situ,* c'est-à-dire au sein du fermenteur.

On connaît par ailleurs le document US 8,945,891 qui divulgue un schéma de récupération de métabolites issus d'une fermentation ABE par injection de gaz dans le bioréacteur suivi d'une étape de récupération du métabolite par absorption avec une composition comprenant de l'isophorone comme solvant.

US 8,460,439 décrit une méthode de récupération du butanol contenu dans un jus fermentaire dans laquelle une partie du jus fermentaire est envoyé à une étape de stripage mettant en oeuvre un gaz inerte de manière à récupérer un gaz enrichi en butanol qui est ensuite traité dans une section d'absorption en présence d'un solvant organique, par exemple un alcool ayant au moins 8 atomes de carbone.

Il est connu de la publication « High-titer n-butanol production by Clostridium acetobutylicum JB200 in fed-batch fermentation with intermittent gas stripping », de Chuang Xue et Al. (Biotechnology and Bioengineering, vol. 109, no. 11, 2012-11-08), un procédé de fermentation de type ABE où, pour récupérer une teneur élevée en butanol, on réalise un strippage au gaz intermittent.

Il est connu de la publication "Prolonged conversion of n-butyrate to n-butanol with Clostridium saccharoperbutylacetonicum in a two-stage continuous culture with in-situ product removal", de Hanno Richter et Al. (Biotechnology and Bioengineering, vol.109, no 4, 2012-04-01), de produire du n-butanol en continu avec un fermenteur à deux étapes, le n-butanol étant récupéré en continu pendant la deuxième étape par un strippage au gaz.

Un but de l'invention est de proposer un procédé alternatif de récupération de produits fermentaires présents dans un moût de fermentation d'un bioréacteur qui soit efficace, simple à mettre en oeuvre et pour lequel les dépenses d'investissement (CAPEX) et d'exploitation (OPEX) soient optimisés.

### Résumé de l'invention

La présente invention concerne donc un procédé de récupération de produits fermentaires présents dans un jus aqueux de fermentation produit dans un bioréacteur qui comprend une étape a) dans laquelle on envoie dans le jus aqueux de fermentation un flux gazeux sous pression de manière à entrainer au moins une partie des alcools et produire un flux gazeux enrichi en alcools. Le procédé selon l'invention comprend une étape b) de stockage préalable des gaz de fermentation produits dans le bioréacteur qui constituent ainsi le flux gazeux qui est envoyé dans le bioréacteur afin d'entrainer les produits fermentaires.

Plus précisément, l'invention selon l'invention a pour objet un procédé de récupération de produits fermentaires présents dans un moût de fermentation produit dans un bioréacteur, comprenant
- une étape a) dans laquelle on envoie dans le moût de fermentation un flux gazeux sous pression de manière à entrainer au moins une partie des produits et produire un flux gazeux enrichi en produits fermentaires,
tel que le procédé comprend :
- une étape b) de stockage des gaz de fermentation produits dans le bioréacteur, en ce que le flux gazeux qui est envoyé à l'étape a) est constitué par les gaz de fermentation stockés
- et une étape c) dans laquelle on met en contact le flux gazeux enrichi en produits fermentaires issu de l'étape a) avec un solvant de façon à produire un solvant enrichi en produits fermentaires.

Le procédé selon l'invention valorise ainsi les gaz produits par la fermentation, que l'on peut considérer comme étant des sous-produits de la fermentation, comme gaz de stripage permettant d'extraire en dehors du système fermentaire les produits d'intérêt. La mise en oeuvre du procédé ne nécessite ainsi plus l'apport de gaz externe au procédé qui génère des coûts de transport non négligeables. Enfin en termes d'investissement, le procédé ne requiert qu'un dispositif (ballon) de stockage qui est toutefois déjà nécessaire dans le cas où le gaz de stripage n'est pas produit *in situ.*

Le procédé selon l'invention présente en outre l'avantage de permettre la récupération d'au moins une partie des produits fermentaires par une méthode autre que la distillation, cette dernière étant particulièrement énergivore en raison du caractère dilué des produits présents dans le moût de fermentation.

Le procédé de récupération *in situ* des produits fermentaires permet par ailleurs un meilleur contrôle de leur teneur dans le milieu fermentaire afin de limiter cette teneur à une valeur seuil qui reste acceptable pour le microorganisme. En effet, il est connu qu'à partir d'une certaine teneur dans le milieu de fermentation les produits fermentaires, et particulièrement les alcools (e.g. le butanol), ont un effet inhibiteur pour le microorganisme.

Le procédé selon l'invention peut être opéré selon un mode dans lequel une fraction du moût de fermentation est soutirée du bioréacteur, l'étape a) étant réalisée en dehors du bioréacteur sur ladite fraction et dans lequel on recycle dans le bioréacteur au moins une partie du moût de fermentation appauvri en produits fermentaires. Alternativement, l'étape a) est réalisée *in situ* dans le bioréacteur.

Afin de récupérer les produits fermentaires présents dans le flux gazeux, le procédé comprend une étape c) dans laquelle le flux gazeux enrichi en produits fermentaires issu de l'étape a) est mis en contact avec un solvant de façon à produire un solvant enrichi en produits fermentaires. L'étape c) peut consister à envoyer le flux gazeux enrichi en produits fermentaires issu de l'étape a) dans une section d'absorption dans laquelle on met en contact ledit flux gazeux avec le solvant organique de manière à récupérer un flux gazeux appauvri en produits fermentaires et un solvant enrichi en produits fermentaires. Avantageusement le flux gazeux appauvri en en produits fermentaires est au moins en partie renvoyé à l'étape b) de stockage.

Selon un autre mode de réalisation, l'étape c) est réalisée dans le bioréacteur qui contient le jus de fermentation et un solvant organique non miscible à l'eau formant une phase organique surnageant le moût de fermentation de sorte que le flux gazeux enrichi en produits fermentaires est mis en contact dans le bioréacteur avec le solvant organique de manière à transférer au moins une partie des produits fermentaires dans ledit solvant.

Le solvant organique peut être choisi parmi des hydrocarbures à chaine linéaire ou ramifiée, des composés hydrocarbures aromatiques, des acides carboxyliques, des alcools ou des esters.

Le procédé selon l'invention peut comprendre une étape d) dans laquelle on récupère le solvant organique enrichi en produits fermentaires et on régénère le solvant enrichi en produits fermentaires de manière à séparer lesdits produits fermentaires et produire un solvant régénéré. De manière préférée, on recycle le solvant régénéré à l'étape c).

Le procédé selon l'invention s'applique avantageusement à un moût de fermentation contenant des produits fermentaires choisis parmi les esters, les cétones, les aldéhydes, les acides carboxyliques et les alcools, seuls ou en mélange. Par exemple le moût de fermentation contient du butanol, éventuellement en mélange avec de l'acétone et/ou de l'isopropanol et de l'éthanol.

De préférence le flux gazeux utilisé comme gaz de stripage à l'étape a) comprend du dioxyde de carbone éventuellement en mélange avec de l'hydrogène. Selon l'invention, le flux gazeux de stripage constitué par les gaz fermentaires peut être traité avant d'être envoyé dans le bioréacteur. Le terme "traité" désigne une étape permettant d'éliminer une partie des composés qui constitue ledit flux gazeux. Dans le cadre de l'invention, le gaz de stripage peut inclure une étape de traitement préalable afin de minimiser sa teneur en hydrogène, par exemple par oxydation ou par combustion de l'hydrogène en milieu oxydant (par exemple en présence d'air, d'oxygène pur, d'oxygène supporté sur un solide agissant comme un catalyseur d'oxydation).

Le procédé selon l'invention peut être mis en oeuvre notamment afin de récupérer des alcools qui peuvent être des produits inhibiteurs de la fermentation. Par exemple le butanol à partir d'une valeur seuil d'environ 10 g/L est un inhibiteur de la fermentation chez *Clostridium.* Selon un mode de réalisation, l'étape a) n'est opérée que lorsque le moût de fermentation a une teneur en produits fermentaires qui est supérieure à une valeur seuil.

L'invention a également pour objet un procédé de production de produits fermentaires, comprenant les étapes suivantes:
i. on fermente dans un bioréacteur une solution aqueuse de sucres en C5 et/ou C6 en présence d'un microorganisme de manière à produire un moût de fermentation contenant des produits fermentaires et des gaz de fermentation;
ii. on envoie du gaz de fermentation produit dans le bioréacteur dans une unité de stockage;
iii. on envoie sous pression, dans le jus aqueux de fermentation, les gaz de fermentation stockés de manière à entrainer les produits fermentaires dans le flux gazeux et produire un flux gazeux enrichi en produits fermentaires;
iv. on met en contact le flux gazeux enrichi en produits fermentaires avec un solvant organique de manière à produire un solvant enrichi en produits fermentaires;
v. on régénère le solvant organique enrichi en produits fermentaires de manière à produire un flux enrichi en produits fermentaires et un solvant régénéré.

Les étapes iii) et iv) peuvent être effectuées dans le bioréacteur ou en dehors du bioréacteur tandis que l'étape v) est effectuée en dehors du bioréacteur.

Selon un mode de réalisation préféré, le moût de fermentation contient du butanol, éventuellement en mélange avec de l'acétone et/ou de l'isopropanol et de l'éthanol.

De préférence le procédé comprend une étape vi) dans laquelle une fraction du moût de fermentation est envoyée dans une section de récupération et séparation des produits fermentaires, ladite section incluant au moins une unité de distillation.

De manière préférée, le flux enrichi en produits fermentaires obtenu à l'étape v) est envoyé dans la section de récupération et de séparation des produits fermentaires.

Le procédé de production de produits fermentaires peut être appliqué lorsque les microorganismes sont immobilisés sur un support dans le bioréacteur.

### Description détaillée de l'invention

Les autres caractéristiques et avantages de l'invention vont apparaître à la lecture de la description qui va suivre, donnée à titre uniquement illustratif et non limitatif, et en référence :
- à la figure 1 qui est un schéma de principe d'un procédé de production d'alcools par fermentation de sucres en C5 et/ou C6 incluant un procédé de récupération partielle des alcools par le procédé selon l'invention;
- à la figure 2 qui montre un schéma de principe d'une unité de séparation des alcools produits par fermentation dans un bioréacteur.

### La charge

Le procédé selon l'invention permet de traiter tout moût de fermentation (ou jus de fermentation) qui comprend une phase aqueuse contenant des produits fermentaires et des microorganismes. Par exemple le moût de fermentation peut contenir un mélange d'alcools ayant au moins deux atomes de carbone.

Le procédé de récupération est applicable à des jus de fermentation obtenus à partir d'une solution aqueuse de sucres en C5 et/ou C6 mise en contact avec des microorganismes anaérobies capables de convertir lesdits sucres en alcools et/ou solvants. De préférence le procédé selon l'invention est utilisé pour traiter des moûts de fermentation produits par des microorganismes du genre *Clostridium* (bactérie de la famille des bacilles gram positives anaérobies). De préférence le microorganisme fermentaire est choisi parmi les souches *Clostridium acetobutylicum* et *Clostridium beijerinckii,* naturelles ou génétiquement modifiée capables de produire des solvants du type ABE (Acétone-Butanol-Ethanol) ou du type IBE (Isopropanol-Butanol-Ethanol).

Pour ces types de fermentation (ABE ou IBE), le procédé permet de façon avantageuse d'extraire, de manière continue ou discontinue, une partie des alcools/ solvants d'intérêt du jus de fermentation. Le procédé permet par ailleurs de limiter la teneur en produits fermentaires dans le milieu de fermentation dont la présence à partir d'une valeur seuil a des effets d'inhibition de la fermentation. Par exemple, dans le cas du butanol cet effet inhibiteur chez *Clostridium* est observé à partir d'une teneur supérieure à 10 g/L.

La solution aqueuse de sucres en C5 et/ou C6 qui est fermentée peut avoir différentes origines. Elle provient de préférence du traitement d'une source renouvelable. De préférence, cette source est du type biomasse lignocellulosique qui comprend notamment les substrats ligneux (feuillus et résineux), les sous-produits de l'agriculture (paille) ou ceux des industries génératrices de déchets lignocellulosiques (industries agroalimentaires, papeteries). La solution aqueuse de sucres peut également être obtenue à partir de plantes sucrières, comme par exemple la betterave sucrière et la canne à sucre ou encore à partir de plantes amylacées comme le maïs ou le blé.

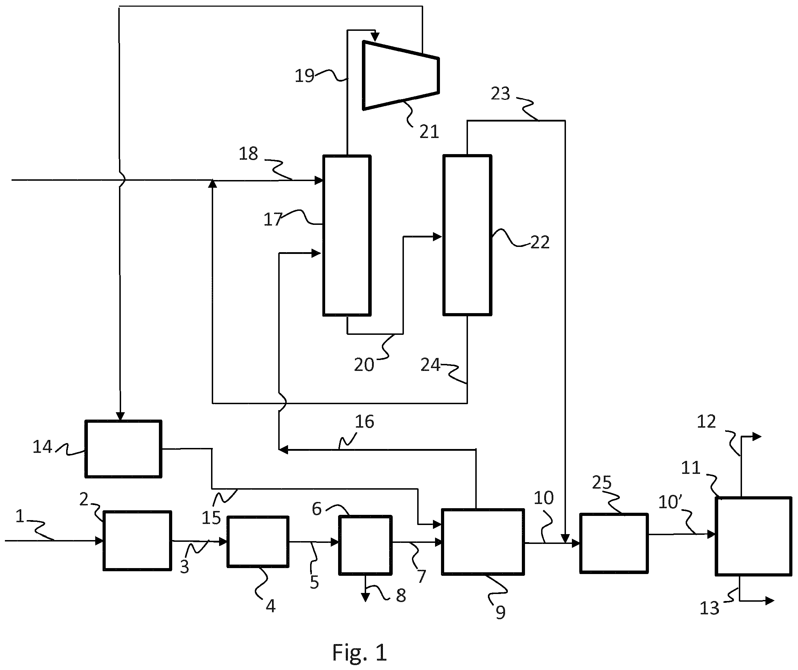

La figure 1 représente un schéma de production de solvants (mélange d'alcools) à partir d'un substrat du type biomasse lignocellulosique.

En référence à la figure 1, une charge de biomasse est amenée dans l'unité de prétraitement 2 par l'intermédiaire du conduit 1. La charge de biomasse peut être composée de bois, des pailles ou de rafles de maïs, de produits de cultures forestières dédiées (par exemple de résineux tels les épicéas ou les pins, ou des feuillus tels les eucalyptus), des plantes de cultures dédiées tells le miscanthus ou le switchgrass, de résidus de plantes alcooligènes, sucrières (par exemple canne à sucre ou betterave) et céréalières (par exemple mais, blé...), de produits et résidus de l'industrie papetière et des produits de transformation des matériaux lignocellulosiques. La charge peut être composée d'environ 35 à 50% poids de cellulose, de 20 à 30% poids d'hémicellulose et de 15 à 25% poids de lignine.

Les composé acide ou basique et l'eau nécessaires au prétraitement sont amenés dans l'unité de prétraitement 2 par l'intermédiaire de conduits (non représentés) afin d'y réaliser une réaction d'hydrolyse en milieu acide ou basique. Dans l'unité 2, la charge de biomasse est mise en contact et mélangée avec l'eau et le composé acide ou basique dans un réacteur. L'unité 2 de prétraitement peut également mettre en oeuvre une action mécanique, créée par exemple au moyen d'une extrudeuse de type bi-vis ou d'une défibreuse. Le composé acide pour le prétraitement peut être choisi parmi de l'acide sulfurique, de l'acide chlorhydrique, de l'acide nitrique, de l'acide acétique ou de l'acide formique. Quant au composé basique, il peut être choisi parmi de l'hydroxyde de potassium, de l'hydroxyde de sodium, de l'ammoniaque.

L'unité de prétraitement peut mettre en oeuvre un procédé AFEX (Ammonia Fiber Explosion) qui consiste à introduire le substrat lignocellulosique dans un cuiseur à haute pression en présence d'ammoniac, puis de provoquer une détente explosive en sortie du réacteur et de recycler l'ammoniac alors sous forme gazeuse. Ce type de procédé est notamment décrit par Teymouri et al., 2005, Biores. Technol. 96 (2005) p.2014-2018. Ce procédé conduit principalement à une déstructuration de la matrice de la biomasse mais il n'y a pas de séparation phasique des composés lignine, hémicellulose et cellulose en sortie de traitement. Selon un deuxième mode de réalisation, on réalise un prétraitement acide dans l'unité 2. Par exemple, on peut mettre en oeuvre un prétraitement de type cuisson à l'acide diluée. Dans ce mode de réalisation la biomasse est mise en contact avec un acide fort dilué dans de l'eau, par exemple l'acide sulfurique, en mettant en oeuvre la biomasse à de faibles teneurs en matières sèche, généralement compris entre 5 et 20% de matière sèche. La biomasse, l'acide et l'eau sont mis en contact dans un réacteur et montés en température, généralement entre 120°C et 200°C. Lors de ce procédé, les composés hémicellulosique sont principalement hydrolysés en sucres, permettant ainsi de déstructurer la matrice lignocellulosique. A l'issue de ce prétraitement acide, on aboutit à la production d'un substrat prétraité solide, enrichi en cellulose et en lignine ainsi qu'une fraction liquide enrichie en sucres.

Selon un troisième mode de réalisation on peut également mettre en oeuvre le procédé nommé "explosion vapeur", ou "SteamEx" ou "Steam Explosion" selon la terminologie anglo-saxonne, dans l'unité 2. C'est un procédé dans lequel la biomasse lignocellulosique est mise en contact avec de l'eau dans un réacteur à faible temps de séjour, généralement compris entre 2 et 15 minutes et à des températures modérées, généralement entre 120°C et 250°C et à une pression comprise entre 0,5 et 5 MPa (5 et 50 bars). L'eau peut être additionnée d'un composé acide, par exemple de l'acide sulfurique, ou d'un composé basique. En sortie du réacteur, la biomasse est détendue, par exemple à pression atmosphérique, dans un récipient séparateur gaz / solide afin de produire une biomasse prétraitée à haute matière sèche, généralement comprise entre 20 et 70% de matière sèche.

On évacue de l'unité de prétraitement 2 un substrat prétraité par le conduit 3. Le substrat prétraité est composé de sucres dissous en phase liquide et de matière solide composée de lignine, de cellulose et d'hémicellulose qui n'a pas été liquéfié dans le prétraitement. Le flux de substrat prétraité circulant dans le conduit 3 contient préférentiellement entre 10% poids et 60% poids de matière sèche et encore plus préférentiellement entre 20% poids et 55% poids de matière sèche.

Le substrat prétraité est introduit dans un réacteur 4 pour subir une étape dite d"hydrolyse enzymatique". De l'eau et des enzymes sont respectivement ajoutés dans le réacteur 4 afin de réaliser une réaction d'hydrolyse enzymatique du substrat prétraité. Les quantités de substrat prétraité d'eau et d'enzyme sont ajustées dans le réacteur d'hydrolyse de manière à ce que le milieu réactionnel comporte une teneur en matière solide généralement comprise entre 5% et 40% poids, de préférence entre 10% et 25% poids. L'hydrolyse enzymatique est préférentiellement réalisée à pH compris entre 4 et 5,5 et à une température comprise entre 35°C et 60°C. Les enzymes peuvent être produites par un microorganisme, par exemples des champignons appartenant aux genres *Trichoderma, Aspergillus, Pénicillium ou Schizophyllum,* ou les bactéries anaérobies appartenant par exemple au genre *Clostridium.* Les enzymes produites par ces microorganismes contiennent notamment les cellulases et éventuellement des hémicellulases, adaptées à réaliser une hydrolyse poussée de la cellulose et éventuellement des hémicelluloses. Les cellulases, respectivement les hémicellulases transforment par hydrolyse la cellulose, respectivement l'hémicellulose, en sucres qui peuvent se dissoudre en phase aqueuse. Dans l'unité d'hydrolyse enzymatique, les conditions opératoires, principalement le taux de matière sèche du mélange à hydrolyser et la quantité d'enzymes utilisée, sont choisies de telle façon que l'on obtienne une solubilisation de la cellulose comprise entre 20% et 99% poids, de préférence entre 30% et 95% poids par rapport au poids total de cellulose contenu dans le substrat prétraité. On évacue du réacteur d'hydrolyse 4 un hydrolysat par le conduit 5. Ainsi l'hydrolysat 5 comprend des sucres dissous en phase aqueuse et de la matière solide composée principalement de lignine, et de cellulose et d'hémicellulose qui n'ont pas été hydrolysées. L'hydrolysat 5 subit ensuite, dans l'unité 6, une étape de séparation entre liquide et solide afin d'en extraire la matière solide, notamment la lignine. La séparation de la matière solide peut mettre en oeuvre l'une des techniques suivantes : centrifugation, essorage ou pressage, filtration, décantation. L'unité 6 produit un flux liquide appauvri en matière solide évacué par le conduit 7 et un flux enrichi en matière solide, notamment en lignine, évacué par le conduit 8.

Le flux aqueux appauvri en solide et contenant des sucres en C5 et/ou C6 est ensuite introduit par le conduit 7 dans une unité de fermentation 9 pour subir une étape de fermentation. Dans l'unité 9, le flux aqueux est alors mis en contact avec un ou plusieurs microorganismes de fermentation. Les microorganismes peuvent être choisis par exemple parmi les éléments suivants : les levures du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis ou* les bactéries *du genre Zymomonas mobilis, Clostridium, Escherichia coli.* Les sucres fermentescibles sont ainsi transformés en alcools et/ou solvants par les microorganismes. L'étape de fermentation dans l'unité 9 peut être réalisée à une température comprise entre 30°C et 37°C de manière à produire un moût (ou jus ou vin) de fermentation contenant des produits de la réaction de fermentation, par exemple des alcools et/ ou des solvants organiques, qui est ensuite évacué par le conduit 10.

Le moût de fermentation est introduit par le conduit 10 dans une unité de séparation 11 permettant de séparer et d'extraire les composés d'intérêt du moût de fermentation, ces derniers étant évacués par le conduit 12. Les résidus de la séparation, couramment appelés vinasses, sont évacués de l'unité de séparation 11 par le conduit 13. Les vinasses sont généralement composées d'eau ainsi que de tout produit liquide ou solide non converti ou non extrait lors des étapes précédentes. L'unité de séparation 11 peut mettre en oeuvre une ou plusieurs distillations, et éventuellement une séparation des matières en suspension par exemple par centrifugation, décantation, filtration.

De préférence le procédé fermentaire est un procédé dit "ABE" ou "IBE" permettant de produire un mélange (Acétone-Butanol-Ethanol) ou (Isopropanol-Butanol-Ethanol) respectivement.

Dans le cadre de l'invention, l'étape de fermentation peut mis en oeuvre soit selon un mode de fonctionnement semi-continu (ou "fed-batch" selon la terminologie anglo-saxonne), soit selon un mode de fonctionnement dit continu qui sont bien connus de l'homme du métier.

Il est à noter que l'étape de fermentation peut être réalisée après l'étape d'hydrolyse enzymatique ou encore simultanément à l'étape d'hydrolyse (fermentation du type "SSF" ou Simultaneous Saccharification and Fermentation selon la terminologie anglo-saxonne).

Enfin il est également possible d'utiliser un bioréacteur dans lequel les microorganismes fermentaires sont immobilisés sur un support.

Selon un mode de réalisation alternatif, le bioréacteur peut comprendre un ensemble incluant un premier réacteur couplé par une première ligne à un second réacteur dans lequel les microorganismes fermentaires sont immobilisés sur un support et une seconde ligne permettant le recyclage du moût fermentaire produit dans le second réacteur dans le premier réacteur. Dans ce mode réalisation, le stripage au gaz fermentaire est effectué au niveau du premier réacteur.

Comme indiqué sur la figure 1, le procédé de production d'alcools met en outre une étape de récupération partielle des alcools produits présents dans le moût (ou vin) de fermentation. Cette étape d'extraction des alcools fait appel à une première étape de stripage avec un gaz sous pression. Comme indiqué dans la figure 1, un gaz sous pression stocké dans un dispositif de stockage 14 (par exemple un ballon) est envoyé par la ligne 15 dans le fermenteur 9 afin d'entrainer les alcools présents dans la phase aqueuse. Généralement le gaz est envoyé sous pression dans le bioréacteur à une vitesse volumique par minute comprise entre 0,5 et 3 L/L/min. Selon une caractéristique importante de l'invention, le gaz de stripage est un gaz produit directement par la fermentation et qui a été préalablement stocké avant sa mise en oeuvre. Le gaz de stripage comprend typiquement du dioxyde de carbone et éventuellement de l'hydrogène. L'étape de récupération met ainsi en jeu un gaz produit *in situ* par l'étape de fermentation et ne nécessite donc pas l'appoint d'un gaz externe audit procédé limitant par conséquent les coûts d'exploitation (OPEX) liés notamment à l'achat et au transport du gaz d'appoint. Dans le cadre de l'invention, le stockage des gaz fermentaires peut se faire avec un (ou plusieurs) gazomètre à cloche scellée par un niveau d'eau. Il est envisageable de réaliser un stockage sous pression avec une recompression préalable, typiquement jusqu'à 6 MPa (60 bars). La détente du gaz utilisé après stockage permet avantageusement de compenser une partie de l'énergie de compression. Le gaz fermentaire peut être également stocké dans des cavités géologiques, du type aquifère lorsque qu'il contient peu d'hydrogène, ce dernier ayant été par exemple brûlé.

Cette étape de stripage au gaz permet avantageusement de contrôler au cours de la fermentation la teneur en alcools présents dans le milieu afin de limiter les phénomènes d'inhibition des microorganismes qui apparaissent lorsque la teneur en alcools atteint une valeur critique. Selon l'invention, cette étape de stripage au gaz peut être soit réalisée en continue soit de manière discontinue. Le débit de gaz fermentaire rapporté au volume de fermenteur est par exemple compris entre 0,5 et 2,5 l/l/min, de préférence compris entre 0,7 et 1,1 l/l/min.

De façon avantageuse, le bioréacteur contient des microorganismes fermentaires qui sont immobilisés sur un support de sorte que lors de l'étape de stripage, les gaz ne soient pas en mesure d'entrainer une partie des microorganismes présents dans le moût de fermentation. En référence à la figure 1, un flux gazeux enrichi en alcools est extrait du fermenteur 9 par la ligne 16, puis est traité dans une étape de séparation afin de récupérer les alcools contenus dans le flux gazeux 16. A cette fin, le flux gazeux est envoyé dans une section d'extraction par solvant mettant en oeuvre au moins une colonne d'absorption 17.

Le flux gazeux 16 est mis en contact avec un solvant ou un mélange de solvant apporté par la ligne 18, de préférence à contre-courant, de manière à produire un flux gazeux purifié à faible teneur en alcools 19 et un flux de solvant enrichi en alcools et à faible teneur en eau 20. Le solvant peut également contenir plusieurs composés. De préférence, le solvant mis en oeuvre dans l'étape d'extraction gaz/liquide a une température d'ébullition supérieure d'au moins 50°C par rapport à celle du produit à récupérer. Le solvant peut être choisi parmi des composés hydrocarbures à chaine linéaire ou ramifiée, des composés hydrocarbures aromatiques, des acides carboxyliques, des alcools ou des esters. Parmi les solvants possibles, on citera les huiles végétales (liquide à partir de 30°C), les alcools à plus de onze atomes de carbone, de préférence β-branchés, les acides à plus de onze atomes de carbone, branchés ou non, de préférence comportant une ou deux fonctions hydroxyle.

Le flux gazeux est mis en contact avec un solvant apporté par la ligne 18, de préférence à contre-courant, de manière à produire un flux gazeux purifié à faible teneur en alcools 19 et un flux de solvant enrichi en alcools et à faible teneur en eau 20.

Comme indiqué sur la figure 1, le flux gazeux purifié 19 est mis sous pression au moyen d'un compresseur 21 et envoyé dans le dispositif de stockage 14 en vue d'être de nouveau envoyé dans le fermenteur 9. Quant au flux de solvant enrichi en alcools 20, il est avantageusement traité dans une unité de régénération du solvant 22 de manière à séparer les alcools de la phase organique. Par exemple cette étape de régénération peut être effectuée par distillation. L'étape de régénération produit un flux d'alcool et un flux de solvant régénéré qui sont évacués de l'unité de régénération 22 par les lignes 23 et 24 respectivement. Le solvant régénéré est par la suite recyclé à la section d'extraction par solvant.

Le flux concentré d'alcools 23 qui est par ailleurs à plus faible teneur en eau que le moût de fermentation (typiquement de l'ordre de 50% poids) est avantageusement mélangé dans une zone de mélange 25 avec le moût 10 soutiré du bioréacteur 9. Ledit mélangé est alors traité dans l'unité de séparation des alcools 11. Par rapport à un schéma classique dans lequel seul le moût de fermentation est traité dans l'unité de séparation des alcools 11, l'étape de séparation du mélange (moût de fermentation + flux concentré en alcools) dans l'unité de séparation des alcools 11 requiert une consommation en énergie plus faible à iso-taux de récupération des alcools produits dans la mesure où le flux d'alcools traité par ladite section contient une teneur plus faible en eau.

Selon un autre mode de réalisation, le procédé de récupération selon l'invention peut également être mis en oeuvre de telle sorte que l'étape de stripage au gaz soit opérée dans un bioréacteur 9 contenant un solvant organique non miscible à l'eau, le solvant formant une phase organique surnageant au-dessus du moût de fermentation. Le solvant sera par ailleurs choisi de manière à être biocompatible avec le microorganisme.

Le gaz de stripage est ainsi injecté dans le moût de fermentation de manière à entrainer les alcools produits dans la phase organique surnageante et de telle façon qu'une partie des alcools soient transférés dans la phase organique lorsque le gaz de stripage traverse ladite phase organique. Le gaz de stripage qui contient encore des alcools est soutiré du fermenteur 9 et est traité dans une unité d'absorption par solvant 17 comme décrit ci-dessus. Lorsque la phase organique contenue dans le fermenteur 9 est saturée en alcools, elle est soutirée et envoyée dans l'unité de régénération du solvant 22 afin de récupérer les alcools et fournir un solvant régénéré qui est recyclé dans le fermenteur 9. Ce mode de mise en oeuvre ne requiert donc pas de boucle de régénération en continue du solvant. Pour ce qui concerne la phase organique enrichie en alcools 20 issue de l'unité d'absorption, celle-ci est avantageusement régénérée dans la même unité de régénération du solvant 22.

Ce mode de fonctionnement améliore la récupération des alcools contenus dans le moût de fermentation étant donné que les produits entrainés par le gaz de stripage sont immédiatement mis en contact avec la phase organique.

Par ailleurs lorsque le procédé de récupération des produits fermentaires selon l'invention vise essentiellement à contrôler la teneur des produits dans le milieu fermentaire en vue de limiter les effets d'inhibition sur le microorganisme, ce mode de réalisation permet notamment de réduire le temps total de stripage au gaz lorsque la quantité de solvant et/ou le pouvoir d'absorption et/ou la sélectivité du solvant (ou mélange de solvants) introduit dans le bioréacteur sont choisis de telle sorte que la valeur seuil de concentration en produits dans la phase aqueuse ne soit atteinte qu'après la saturation de la phase organique.

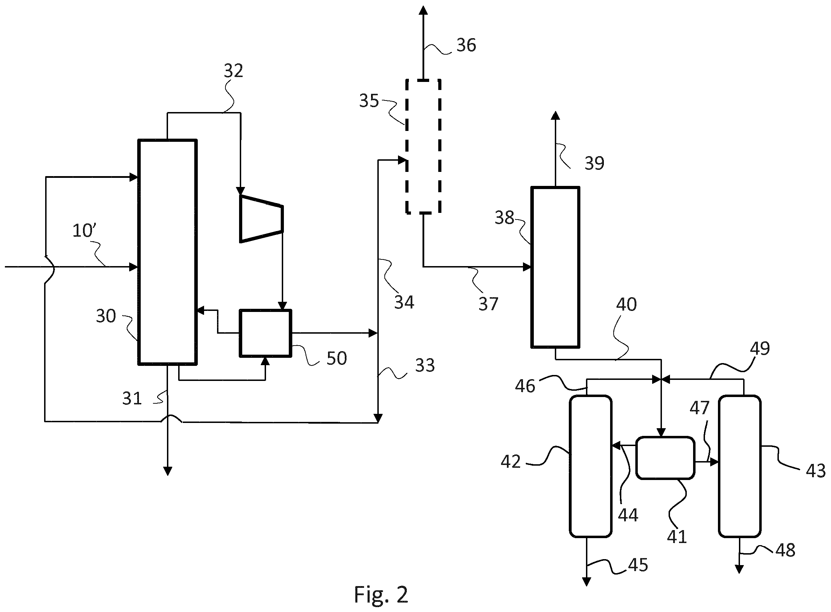

La figure 2 montre un schéma de principe d'une unité de séparation 11 des alcools produits par une fermentation du type IBE, c'est-à-dire un mélange Isopropanol-Butanol-Ethanol avec éventuellement de l'acétone.

Conformément à un mode réalisation préféré du procédé de production de produits fermentaires selon l'invention, le mélange aqueux 10' comprenant le moût de fermentation 10 soutiré du bioréacteur et le flux enrichi en alcools 23 issu de l'unité de régénération du solvant 22 est traité dans l'unité de séparation des alcools 11. En référence à la figure 2, le mélange 10' est envoyé dans une première colonne de distillation 30, également appelée "colonne à bière". La colonne 30 est conçue pour séparer une partie de l'eau 31 contenue dans le mélange qui est récupérée en fond de ladite colonne. La composition de cette eau est telle qu'elle peut en partie être directement recyclée en amont du bioréacteur, l'autre partie étant envoyée au traitement des eaux, avant d'être elle aussi recyclée en amont du fermenteur. En tête de la colonne 30 est soutiré un mélange aqueux enrichi en alcools 32 (IBE avec éventuellement de l'acétone). Le flux récupéré en tête de cette première colonne est plus concentrée en alcools que la charge. Il est par exemple possible d'atteindre un facteur de concentration en alcools supérieur ou égal à 25 (g.l⁻¹ par g.l⁻¹).

De préférence comme représenté à la figure 2, la colonne 30 met en oeuvre un système de rebouillage 50 par recompression mécanique des vapeurs de tête. Ce système permet d'abaisser la demande énergétique de cette colonne d'environ 30 à 50%.

Le flux récupéré en tête de cette première colonne 30 est en partie recyclé dans la colonne 30 en tant que reflux via la ligne 33. L'autre partie du flux non recyclée 34 est éventuellement envoyé dans une deuxième colonne de distillation 35. Le rôle de cette deuxième colonne 35 est de séparer l'acétone du flux d'alcools, l'acétone étant extrait en tête de la colonne 35 par la ligne 36, et de produire un flux aqueux concentré en isopropanol-butanol-éthanol qui est soutiré en fond par la ligne 37.

Le flux 37 est ensuite envoyé dans une troisième colonne de distillation 38 conçue et opérée pour séparer un mélange de tête 39 contenant éthanol/isopropanol/eau de composition azéotropique et un effluent aqueux de fond 40 concentré en butanol. Afin de gérer les phénomènes de démixtion qui peuvent apparaître à partir d'une certaine concentration de butanol, la colonne 38 est de préférence équipée d'une ou plusieurs zones de démixtion liquide/liquide/vapeur comportant des internes spécifiques. Alternativement, on peut opérer la colonne 38 à une pression légèrement plus élevée afin de supprimer ces phénomènes de démixtion.

En fonction de la teneur en eau du mélange traitée 10' et de la teneur en eau de mélange azéotrope éthanol/isopropanol/eau 39 produit en tête de la troisième colonne 38, la teneur en eau du flux 40 est plus ou moins élevée. S'il est nécessaire de sécher le flux aqueux de butanol 40, celui-ci peut être traité par un système de distillation hétéroazéotropique.

Comme représenté sur la figure 2, le flux aqueux de butanol 40 est envoyé dans une unité de démixtion du butanol afin de récupérer le butanol. A cette fin, le flux aqueux de butanol est refroidi, par exemple à une température de 60°C dans un ballon séparateur 41 afin de démixer le mélange en deux phases à savoir une phase organique contenant essentiellement du butanol (par exemple au moins 70% poids de butanol) et une phase aqueuse.

Les deux phases sont traitées dans un système de distillation hétéroazéotropique qui comprend deux colonnes 42, 43 fonctionnant en parallèle. La phase organique contenant majoritairement du butanol est envoyée par la ligne 44 dans la colonne de distillation hétéroazéotropique 42 qui fonctionne par exemple à une pression comprise entre 0,3 et 10 MPa et à une température comprise entre 115 et 150°C afin d'éviter des problèmes de démixtion liquide-liquide-vapeur. On soutire en fond de ladite colonne 42 par la ligne 45 un effluent ayant une teneur massique d'au moins 99% en butanol et en tête, par la ligne 46, un effluent aqueux qui est renvoyé dans le ballon séparateur 41.

La phase aqueuse contenant encore du butanol, qui est soutirée du ballon séparateur biphasique 41 par la ligne 47 dans la colonne de distillation hétéroazéotropique 43. De ladite colonne 43 on récupère respectivement en fond un flux riche en eau 48 et en tête un effluent contenant du butanol qui est recyclé, via la ligne 49, dans le ballon séparateur biphasique 41. La colonne 43 est opérée dans des conditions moins sévères, par exemple à une pression inférieure à celle de la colonne 42.

### Exemple

L'exemple ci-dessous est construit par simulation en utilisant un logiciel de conception et d'analyse opérationnelle de processus (Simsci Pro/ll) qui intègre des résultats issus de tests laboratoire et issus de la littérature concernant la fermentation au moyen de microorganismes du genre *Clostridium.*

Pour simulation, il a été supposé que l'unité de production fermentaire met en oeuvre une unité de fermentation comprenant dix fermenteurs qui traite une solution aqueuse de glucose à 50 g/L. Le volume total de l'unité de fermentation est de 10*500 m³ et avec un volume utile total de 10*400 m³. En partant de l'hypothèse que la productivité totale est de 0,54 g/l/h de solvant avec une répartition en pourcentage massique en Isopropanol/Butanol/Ethanol: 28%/62%/10%). L'unité de production produit ainsi 17 000 t/an de mélange IBE, soit environ 4 800 t/an d'isopropanol, 10 560 t/an de butanol et 1600 t/an d'éthanol.

La consommation de sucre de l'usine est d'environ 53 000 t/an de glucose en supposant un rendement de 0,32 g de IBE /g de sucre

Les fermenteurs fonctionnent à 37°C selon un mode en batch pendant 34 heures ensuite l'inhibition par le butanol est atteint.

La composition finale du moût de fermentation issu des fermenteurs est la suivante : 4,8 g/l d'isopropanol, 10,5 g/l de butanol et 1,7 g/l d'éthanol, soit environ 17 g/l de produits IBE dans le moût fermentaire.

En partant de l'hypothèse que l'énergie nécessaire pour la séparation des alcools contenus dans le moût fermentaire d'une part et pour séparer le butanol du mélange isopropanol + éthanol (directement valorisable en pétrochimie) d'autre part est d'environ 20 MJ/kg de mélange IBE, on évalue la consommation de vapeur à environ 150 000 t/an de vapeur (soit environ 8,8 t de vapeur/t de mélange IBE.

Le processus naturel de fermentation libère 12 000 Nm³/h en supposant que le débit de gaz (en litre par minute) rapporté au volume du fermenteur (en litre) est de 0,05 l/l/min de gaz qui est un mélange comprenant essentiellement du CO₂ et du H₂ qui n'est pas utilisé comme gaz de stripage.

### Exemple selon l'invention

On met en oeuvre le procédé selon l'invention dans la même unité décrite plus haut qui comprend dix fermenteurs IBE.

Les fermenteurs sont soumis à un stripage au gaz de fermentation lorsque la concentration en métabolite inhibiteur en butanol dans le milieu fermentaire est à moins de 80% du seuil d'inhibition (fixé à 10 g/l de butanol). Le stripage est réalisé avec un débit de gaz (en litre par minute) rapporté au volume du fermenteur (en litre) de 1 l/l/min, soit 240 000 Nm3/h de gaz de fermentation. Ce gaz de fermentation a été préalablement stocké par exemple dès le démarrage de la fermentation afin de disposer d'un volume suffisant.

Afin de limiter le phénomène d'à-coups lors de l'injection du gaz de fermentation pour le stripage, il est préférable de disposer d'une réserve en gaz fermentaire qui est équivalent à au moins 3 minutes d'injection pour un débit donné
Du gaz fermentaire continue à être produit à un débit de 12 000 Nm3/h et est stocké au niveau de l'unité de stockage et éventuellement purgé lorsque le gaz fermentaire est en excédent par rapport à la quantité nécessaire pour le stripage. Le gaz fermentaire de stripage contenant les alcools est envoyé dans une unité de séparation des alcools qui met en oeuvre un contactage dudit gaz avec une huile végétale liquide à 37°C (par exemple de colza, palme ou tournesol). Les effluents produits par l'étape de séparation des alcools sont une l'huile végétale chargée en alcools et un gaz de fermentation appauvri en alcools. Ce dernier est avantageusement recyclé comme gaz de stripage. L'huile végétale contenant les alcools produits est envoyée dans une colonne de distillation opérée sous vide afin de ne pas dégrader thermiquement l'huile. En fond de cette colonne on récupère l'huile épurée des alcools et en tête un mélange aqueux d'alcools. Ce flux concentré en alcools est au final mélangé avec le moût restant dans les fermenteurs en fin de fermentation.

L'extraction par stripage avec le gaz fermentaire du produit le plus inhibiteur, c'est-à-dire dans le cas présent le butanol, a plusieurs effets :

### 1. Sur la productivité

Etant donné qu'on élimine au fur et à mesure le métabolite le plus inhibiteur du moût fermentaire, la souche *Clostridium* augmente sa productivité globale qui est de l'ordre à 0,94 g d'IBE/l/h. On peut alors produire environ 30 000 t/an de mélange IBE, soit environ 7 300 t/an d'isopropanol, 20 500 t/an de butanol et 2 200 t/an d'éthanol. On augmente la production de l'unité d'environ 75%.

### 2. Sur le rendement

On a besoin de moins de sucre en relatif pour augmenter la production. Le rendement est estimé à 0,4 g IBE/g glucose (soit une augmentation de 25% du rendement), ce qui implique une consommation de 75 000 t/an de glucose.

### 3. Energie de séparation

Le moût fermentaire final contient toujours environ 17 g/l de mélange IBE, avec environ 4 g/L d'isopropanol, 11,6 g/l de butanol et 1,4 g/l d'éthanol. Cependant, 90% des alcools produits sont récupérés par les étapes de stripage, de contactage avec un solvant et de régénération du solvant. Le fait d'envoyer les alcools issus de l'étape de régénération dans le moût final récupéré des fermenteurs permet de réduire notablement les coûts énergétiques liés à la séparation finale du butanol. Ainsi en tenant compte de l'énergie de distillation de l'huile végétale d'une part (2 MJ/kg de mélange IBE) et la séparation du butanol du mélange isopropanol + éthanol (5 MJ/kg de mélange IBE) d'autre part, on estime la consommation en vapeur à environ 93 000 t/an de vapeur, soit environ 3,1 t vapeur/t de mélange IBE, d'où une réduction d'environ 65% de l'énergie.

### 4. Rallongement du temps de cycle

En raison de la levée d'inhibition par l'élimination du butanol du moût, on peut opérer les fermentations en mode fed-batch ou continu, ce qui a pour avantage d'allonger le temps d'opération d'environ 500 heures.

## Revendications

1. Procédé de récupération de produits fermentaires présents dans un moût de fermentation produit dans un bioréacteur, comprenant
- une étape a) dans laquelle on envoie dans le moût de fermentation un flux gazeux sous pression de manière à entrainer au moins une partie des produits et produire un flux gazeux enrichi en produits fermentaires,
**caractérisé en ce que** le procédé comprend :
- une étape b) de stockage des gaz de fermentation produits dans le bioréacteur, **en ce que** le flux gazeux qui est envoyé à l'étape a) est constitué par les gaz de fermentation stockés
- et une étape c) dans laquelle on met en contact le flux gazeux enrichi en produits fermentaires issu de l'étape a) avec un solvant de façon à produire un solvant enrichi en produits fermentaires.

2. Procédé selon la revendication 1, dans lequel une fraction du moût de fermentation est soutirée du bioréacteur, l'étape a) est réalisée en dehors du bioréacteur sur ladite fraction du moût de fermentation et dans lequel on recycle dans le bioréacteur au moins une partie du moût de fermentation appauvri en produits fermentaires.

3. Procédé selon la revendication 1, dans lequel l'étape a) est directement réalisée dans le bioréacteur.

4. Procédé selon l'une des revendications précédentes, dans lequel on envoie le flux gazeux enrichi en produits fermentaires issu de l'étape a) dans une section d'absorption gaz/liquide dans laquelle on met en contact ledit flux gazeux avec le solvant organique de manière à produire un solvant enrichi en produits fermentaires et un flux gazeux appauvri en produits fermentaires.

5. Procédé selon l'une des revendications 1 à 3, dans lequel l'étape c) réalisée dans le bioréacteur comprend en outre un solvant organique non miscible à l'eau formant une phase organique surnageant le moût de fermentation, de sorte que l'on met en contact le flux gazeux enrichi en produits fermentaires dans le bioréacteur de manière à transférer au moins une partie des produits fermentaires dans ledit solvant.

6. Procédé selon les revendications précédentes, qui comprend une étape d) dans laquelle on régénère le solvant enrichi en produits fermentaires de manière à séparer lesdits produits fermentaires et produire un solvant régénéré.

7. Procédé selon l'une des revendications précédentes, dans lequel le moût de fermentation contient des produits fermentaires choisis parmi les esters, les cétones, les aldéhydes, les acides carboxyliques et les alcools, seuls ou en mélange.

8. Procédé selon la revendication précédente, dans lequel le moût de fermentation contient du butanol, éventuellement en mélange avec de l'acétone et/ou de l'isopropanol et de l'éthanol.

9. Procédé selon l'une des revendications précédentes, dans lequel le flux gazeux comprend du dioxyde de carbone éventuellement en mélange avec de l'hydrogène.

10. Procédé selon l'une des revendications précédentes, dans lequel le flux gazeux est traité avant d'être envoyé dans le bioréacteur.

11. Procédé selon l'une des revendications précédentes, dans lequel le solvant organique est choisi parmi des hydrocarbures à chaine linéaire ou ramifiée, des composés hydrocarbures aromatiques, des acides carboxyliques, des alcools ou des esters.

12. Procédé de production de produits fermentaires, comprenant les étapes suivantes:
i. on fermente dans un bioréacteur une solution aqueuse de sucres en C5 et/ou C6 en présence d'un microorganisme de manière à produire un moût de fermentation contenant produits fermentaires et des gaz de fermentation;
ii. on envoie du gaz de fermentation produit dans le bioréacteur dans une unité de stockage;
iii. on envoie sous pression, dans le jus aqueux de fermentation, les gaz de fermentation stockés de manière à entrainer les produits fermentaires dans le flux gazeux et produire un flux gazeux enrichi en produits fermentaires;
iv. on met en contact le flux gazeux enrichi en produits fermentaires avec un solvant organique de manière à produire un solvant enrichi en produits fermentaires;
v. on régénère le solvant organique enrichi en produits fermentaires de manière à produire un flux enrichi en produits fermentaires et un solvant régénéré.

13. Procédé selon la revendication 12, dans lequel les étapes iii) et iv) sont effectuées dans le bioréacteur ou en dehors du bioréacteur et dans lequel l'étape v) est effectuée en dehors du bioréacteur.

14. Procédé selon l'une des revendications 12 ou 13, dans lequel le moût de fermentation contient du butanol, éventuellement en mélange avec de l'acétone et/ou de l'isopropanol et de l'éthanol.

15. Procédé selon l'une des revendications 12 à 14, comprenant en outre une étape vi) dans laquelle une fraction du moût de fermentation est envoyée dans une section de récupération et séparation des produits fermentaires, ladite section incluant au moins une unité de distillation.

16. Procédé selon la revendication 15, dans lequel le flux enrichi en produits fermentaires obtenu à l'étape v) est envoyé en mélange avec la fraction de moût de fermentation dans la section de récupération et de séparation des produits fermentaires.

17. Procédé selon l'une des revendications 12 à 16, dans lequel les microorganismes sont immobilisés sur un support dans le bioréacteur.

## Patentansprüche

1. Verfahren zur Gewinnung von Gärungsprodukten, die in einer Gärungsmaische vorliegen, welche in einem Bioreaktor erzeugt wurde, umfassend
- einen Schritt a), in welchem ein Gasstrom derart unter Druck in die Gärungsmaische eingeleitet wird, dass mindestens ein Teil der Produkte mitgerissen wird und ein Gasstrom erzeugt wird, der an Gärungsprodukten angereichert ist,
**dadurch gekennzeichnet, dass** das Verfahren Folgendes umfasst:
- einen Schritt b) des Speicherns der Gärungsgase, welche in dem Bioreaktor erzeugt wurden, wobei der Gasstrom, welcher in Schritt a) eingeleitet wird, aus den gespeicherten Gärungsgasen besteht
und einen Schritt c), in welchem der Gasstrom, welcher an Gärungsprodukten angereichert ist und aus dem Schritt a) stammt, derart mit einem Lösungsmittel in Kontakt gebracht wird, dass ein Lösungsmittel erzeugt wird, das an Gärungsprodukten angereichert ist.

2. Verfahren nach Anspruch 1, wobei eine Teilmenge der Gärungsmaische aus dem Bioreaktor abgezogen wird, der Schritt a) außerhalb des Bioreaktors an der Teilmenge der Gärungsmaische vorgenommen wird und wobei mindestens ein Teil der Gärungsmaische, welche an Gärungsprodukten abgereichert ist, in den Bioreaktor zurückgeführt wird.

3. Verfahren nach Anspruch 1, wobei der Schritt a) direkt in dem Bioreaktor vorgenommen wird.

4. Verfahren nach einem der vorhergehenden Ansprüche, wobei der Gasstrom, welcher an Gärungsprodukten angereichert ist und aus dem Schritt a) stammt, in einen Gas/Flüssigkeits-Absorptionsabschnitt eingeleitet wird, in welchem der Gasstrom derart mit dem organischen Lösungsmittel in Kontakt gebracht wird, dass ein Lösungsmittel, welches an Gärungsprodukten angereichert ist, und ein Gasstrom, welcher an Gärungsprodukten abgereichert ist, erzeugt werden.

5. Verfahren nach einem der Ansprüche 1 bis 3, wobei der Schritt c), welcher in dem Bioreaktor vorgenommen wird, darüber hinaus ein organisches Lösungsmittel umfasst, das nicht mit Wasser mischbar ist und eine organische Phase bildet, welche derart auf der Gärungsmaische aufschwimmt, dass der Gasstrom, welcher an Gärungsprodukten angereichert ist, im Bioreaktor derart in Kontakt gebracht wird, dass zumindest ein Teil der Gärungsprodukte in das Lösungsmittel übergeht.

6. Verfahren nach den vorhergehenden Ansprüchen, wobei es einen Schritt d) umfasst, in welchem das Lösungsmittel, welches an Gärungsprodukten angereichert ist, derart regeneriert wird, dass die Gärungsprodukte abgetrennt werden und ein regeneriertes Lösungsmittel erzeugt wird.

7. Verfahren nach einem der vorhergehenden Ansprüche, wobei die Gärungsmaische Gärungsprodukte enthält, die aus den Estern, Ketonen, Aldehyden, Carbonsäuren und Alkoholen, für sich genommen oder in Mischung, ausgewählt sind.

8. Verfahren nach dem vorhergehenden Anspruch, wobei die Gärungsmaische Butanol, möglicherweise in Mischung mit Aceton und/oder Isopropanol und Ethanol, enthält.

9. Verfahren nach einem der vorhergehenden Ansprüche, wobei der Gasstrom Kohlendioxid, möglicherweise in Mischung mit Wasserstoff, umfasst.

10. Verfahren nach einem der vorhergehenden Ansprüche, wobei der Gasstrom behandelt wird, bevor er in den Bioreaktor eingeleitet wird.

11. Verfahren nach einem beliebigen der vorhergehenden Ansprüche, wobei das organische Lösungsmittel aus den geradkettigen oder verzweigten Kohlenwasserstoffen, den aromatischen Kohlenwasserstoffverbindungen, den Carbonsäuren, Alkoholen oder Estern ausgewählt ist.

12. Verfahren zur Herstellung von Gärungsprodukten, das die folgenden Schritte umfasst:
i. Vergären, in einem Bioreaktor, einer wässrigen Lösung von C5- und/oder C6-Zuckern in Gegenwart eines Mikroorganismus, sodass eine Gärungsmaische entsteht, die Gärungsprodukte und Gärungsgase enthält;
ii. Einleiten des Gärungsgases, welches in dem Bioreaktor erzeugt wird, in eine Speichereinheit;
iii. Einleiten, unter Druck, der gespeicherten Gärungsgase in die wässrige Gärungsflüssigkeit, sodass die Gärungsprodukt in den Gasstrom mitgerissen werden und ein Gasstrom erzeugt wird, der an Gärungsprodukten angereichert ist;
iv. Inkontaktbringen des Gasstroms, welcher an Gärungsprodukten angereichert ist, mit einem organischen Lösungsmittel, sodass ein Lösungsmittel erzeugt wird, das an Gärungsprodukten angereichert ist;
v. Regenerieren des organischen Lösungsmittels, welches an Gärungsprodukten angereichert ist, sodass ein Stoffstrom, der an Gärungsprodukten angereichert ist, und ein regeneriertes Lösungsmittel erzeugt werden.

13. Verfahren nach Anspruch 12, wobei die Schritte iii) und iv) in dem Bioreaktor oder außerhalb des Bioreaktors vorgenommen werden und wobei der Schritt v) außerhalb des Bioreaktors vorgenommen wird.

14. Verfahren nach einem der Ansprüche 12 oder 13, wobei die Gärungsmaische Butanol, möglicherweise in Mischung mit Aceton und/oder Isopropanol und Ethanol, enthält.

15. Verfahren nach einem der Ansprüche 12 bis 14, wobei es darüber hinaus einen Schritt vi) umfasst, in welchem eine Teilmenge der Gärungsmaische in einen Abschnitt zum Gewinnen und Abtrennen der Gärungsprodukte eingeleitet wird, wobei der Abschnitt mindestens eine Destillationseinheit beinhaltet.

16. Verfahren nach Anspruch 15, wobei der Stoffstrom, welcher an Gärungsprodukten angereichert ist und in Schritt v) erhalten wurde, in Mischung mit der Teilmenge der Gärungsmaische in den Abschnitt zum Gewinnen und Abtrennen der Gärungsprodukte eingeleitet wird.

17. Verfahren nach einem der Ansprüche 12 bis 16, wobei die Mikroorganismen im Bioreaktor auf einem Träger immobilisiert sind.

## Claims

1. Process for the recovery of fermentative products present in a fermentation must produced in a bioreactor, comprising:
- a stage a) in which a gas stream under pressure is sent into the fermentation must so as to entrain at least a part of the products and to produce a gas stream enriched in fermentative products,
**characterized in that** the process comprises:
- a stage b) of storage of the fermentation gases produced in the bioreactor, **in that** the gas stream which is sent to stage a) consists of the stored fermentation gases,
- and a stage c) in which the gas stream enriched in fermentative products resulting from stage a) is brought into contact with a solvent, so as to produce a solvent enriched in fermentative products.

2. Process according to Claim 1, in which a fraction of the fermentation must is withdrawn from the bioreactor, stage a) is carried out outside the bioreactor on said fraction of the fermentation must and in which at least a part of the fermentation must depleted in fermentative products is recycled in the bioreactor.

3. Process according to Claim 1, in which stage a) is carried out directly in the bioreactor.

4. Process according to one of the preceding claims, in which the gas stream enriched in fermentative products resulting from stage a) is sent to a gas/liquid absorption section in which said gas stream is brought into contact with the organic solvent so as to produce a solvent enriched in fermentative products and a gas stream depleted in fermentative products.

5. Process according to one of Claims 1 to 3, in which stage c) carried out in the bioreactor additionally comprises a water-immiscible organic solvent forming an organic phase floating on the fermentation must, so that the gas stream enriched in fermentative products is brought into contact in the bioreactor so as to transfer at least a part of the fermentative products into said solvent.

6. Process according to the preceding claims, which comprises a stage d) in which the solvent enriched in fermentative products is regenerated, so as to separate said fermentative products and to produce a regenerated solvent.

7. Process according to one of the preceding claims, in which the fermentation must contains fermentative products chosen from esters, ketones, aldehydes, carboxylic acids and alcohols, alone or as a mixture.

8. Process according to the preceding claim, in which the fermentation must contains butanol, optionally as a mixture with acetone and/or isopropanol and ethanol.

9. Process according to one of the preceding claims, in which the gas stream comprises carbon dioxide, optionally as a mixture with hydrogen.

10. Process according to one of the preceding claims, in which the gas stream is treated before being sent to the bioreactor.

11. Process according to one of the preceding claims, in which the organic solvent is chosen from linear- or branched-chain hydrocarbons, aromatic hydrocarbon compounds, carboxylic acids, alcohols or esters.

12. Process for the production of fermentative products, comprising the following stages:
i. an aqueous solution of C5 and/or C6 sugars is fermented in a bioreactor in the presence of a microorganism so as to produce a fermentation must containing fermentative products and fermentation gases;
ii. fermentation gas produced in the bioreactor is sent to a storage unit;
iii. the stored fermentation gases are fed under pressure into the aqueous fermentation liquor so as to entrain the fermentative products in the gas stream and to produce a gas stream enriched in fermentative products;
iv. the gas stream enriched in fermentative products is brought into contact with an organic solvent so as to produce a solvent enriched in fermentative products;
v. the organic solvent enriched in fermentative products is regenerated so as to produce a stream enriched in fermentative products and a regenerated solvent.

13. Process according to Claim 12, in which stages iii) and iv) are carried out in the bioreactor or outside the bioreactor and in which stage v) is carried out outside the bioreactor.

14. Process according to either of Claims 12 and 13, in which the fermentation must contains butanol, optionally as a mixture with acetone and/or isopropanol and ethanol.

15. Process according to one of Claims 12 to 14, additionally comprising a stage vi) in which a fraction of the fermentation must is sent to a section for recovery and separation of the fermentative products, said section including at least one distillation unit.

16. Process according to Claim 15, in which the stream enriched in fermentative products which is obtained in stage v) is sent, as a mixture with the fermentation must fraction, to the section for recovery and separation of the fermentative products.

17. Process according to one of Claims 12 to 16, in which the microorganisms are immobilized on a support in the bioreactor.
